# EUROPEAN PATENT SPECIFICATION

(11) **EP 1 438 976 B1**
(45) Date of publication and mention of the grant of the patent: **19.12.2007**
(21) Application number: 01274505.5
(22) Date of filing: 28.09.2001
(51) Int. Cl.: A61L 27/16, A61L 27/52, A61L 27/54

(54) **POLYFUNCTIONAL BIOCOMPATIBLE HYDROGEL AND METHOD FOR THE PRODUCTION THEREOF**
POLYFUNKTIONALES BIOKOMPATIBLES HYDROGEL UND HERSTELLUNGSVERFAHREN DAFÜR
HYDROGEL BIOCOMPATIBLE POLYFONCTIONNEL ET PROCEDE DE PRODUCTION ASSOCIE

(43) Date of publication of application: 21.07.2004
(73) Proprietor: Biopharma Development Ltd, London NW11 7TJ (GB)
(72) Inventor: LOPATIN, Vladislav Victorovich, Moscow, 119270 (RU)
(74) Representative: Schubert, Klemens
(86) International application number: PCT/RU2001/000392
(87) International publication number: WO 2003/026711

(56) References cited:
- EP-A2- 0 727 232
- WO-A1-99/10021
- RU-C1- 2 067 873
- RU-C1- 2 127 095
- US-A- 5 941 909
- DATABASE WPI Week 199826 Derwent Publications Ltd., London, GB; AN 1998-295476 XP002423139 & RU 2 088 234 C1 (AS USSR HIGH MOL WT CPDS INST) 27 August 1997 (1997-08-27)

## Description

### Field of the Invention

This invention relates to the formula and method of production of a biocompatible hydrogel, based on the cross-linked copolymer of acrylamide with cross-linking agents. This gel can be used for medical purposes, for example:
- at endoprosthesis replacement by way of purposeful hydrogel injection for the plasty of facial soft-tissues, mastoplasty, phalloplasty, plasty of gastrocnemius muscles, vocal cords and other tissues, which density corresponds to the density of the hydrogel;
- as a filling compound at manufacturing of endoprosthesis, including mammary endoprosthesis;
- as a depot for drugs during long pharmacotherapy, for example, during treatment of tumours or abscesses;
- as a carrier for cultivation of human and animal cells with the subsequent implantation of the hydrogel, which contains the above-mentioned cells, into the bodies of mammals.

### State of the Art

Medical practice is faced with the task to produce artificial materials for replacement of soft tissues - muscular and subcutaneous tissues. They must be rather cheap and easy-to-make, posses all necessary physical and chemical (certain density and chemical inertness, ability to shrink or swell, being placed into the body) and biological (biological inertness, lack of rejection or any other tissue response, in particular) characteristics. Besides, the material must have the form, which is suitable for introduction into muscular tissue with minimum injuries for the patient's organism.

Hydrogels on polyacrylamide bases can be used for such purposes.

There is data on the hydrogel, based on copolymer of acrylamide with methylene-bis-acrylamide as a cross-linking agent. It is described in patent GB No. 2114578. It is meant for production of lens and contains 11,0 mass% of copolymer of acrylamide and methylene-bis-acrylamide, taken in mass ratio of 100 : 2,26, and 89 mass% of a physiologic salt solution.

The method of production of such hydrogel, described in the same patent (GB No. 2114578) consists in the process of copolymerization of acrylamide and methylene-bis-acrylamide, dissolved in the physiologic salt solution in the presence of polymerization initiators, one of which is tetramethylethylenediamine, with the subsequent washing of the end hydrogel from unreacted monomers. Copolymerization reaction is carried out in one stage at room temperature.

However, the hydrogel, received this way, is not suitable for utilization at soft-tissue plasty due to its high density. Besides, on account of copolymerization, carried out in one stage, such gel contains a great number of free radicals and monomers, which exert negative influence on the organism's tissue response.

There is also data on a biocompatible hydrogel, described in application EP No.742022. It contains from 3,5 to 9,0 mass% of copolymer of cross-linked acrylamide with cross-linking agents - methylene-bis-acrylamide and 96,5 - 99,0 mass% of water.

This hydrogel is produced by the method, described in the same application (EP No. 742022). It consists in the reaction of copolymerization of acrylamide with methylene-bis-acrylamide in aqueous compound in the presence of peroxide initiators of polymerization. The reaction mixture must be held for 20 minutes at room temperature for linking of copolymer. At that the copolymerization process is carried out in one stage. The mixture of ammonium persulfate and tetramethylethylenediamine is used as peroxide initiator of polymerization. Apyrogenic water or sodium chloride solution is used as aqueous medium.

The hydrogel, received this way, has an inadequate degree of linking, which is caused by low temperature conditions of the copolymerization process and one-staged reaction. This leads to a quick germination of the connective tissue into the implanted gel, its rapid shrinkage and resorption (A.B.Shekhter et all "Injectable hydrophilic polyacrylamide gel Formacryl and tissue response to its implantation", in magazine "Records of Plastic, Reconstructive and Aesthetic Surgery", 1997, N° 2, p.19).

Moreover, the hydrogel, received this way, contains unconnected molecules of tetramethylethylenediamine, free NH2 radicals and acrylamide monomers in the amount of 1,0 - 1,2 mcg for 1 gram of polymer (1,0 - 1,2 ppm). This can provoke an aseptic inflammatory response at an early stage of hydrogel's injection into the body. (A.B.Shekhter et all "Injectable hydrophilic polyacrylamide gel Formacryl and tissue response to its implantation", in magazine "Records of Plastic, Reconstructive and Aesthetic Surgery", 1997, Nº 2, p.19).

There is also data on a biocompatible gel, described in patent RU No. 2127129. It contains from 1,0 to 8,0 mass% of copolymer of cross linked acrylamide with cross-linking agent - methylene-bis-acrylamide and 92,0 - 99,0 mass% of water. The method of its production is also described in patent RU No.2127129. It consists in copolymerization of acrylamide with methylene-bis-acrylamide in aqueous dispersion medium in the presence of polymerization's peroxide initiator. At that water with pH of 9,0 - 9,5, exposed to electrolysis, is taken as an aqueous medium. Linking of copolymer is carried out at incubation of the reaction compound in two stages: at the temperature of 20 - 90°C during 2 - 24 hours and then at the temperature of 100 - 105°C during 2 - 4 hours.

The hydrogel, received this way, contains no tetramethylethylenediamine, a bit more than 1% of free NH2 radicals and acrylamide monomers in the amount of 0,6 - 0,8 mcg for 1 gram of polymer (0,6 - 0,8 ppm). However, after implantation into the patient's organism, there is observed shrinkage of the material up to 12 - 20 % from the original mass, depending on water content. This reduces the cosmetic effect of the plastic operation, and sometimes there is a need for an additional injection of the material. Besides, polyacrylamide hydrogels can, like agar-agar, serve carriers for reproduction of bacteria and it can provoke an inflammatory process in case microflora got into the implant, from the recipient's body as well.

### Disclosure of the Invention

The offered invention is aimed at reduction of the resorption and shrinkage rate of the biocompatible hydrogel, based on cross-linked polyacrylamide, after its implantation into the patient's body.

The second aim is to cut the possibility of pathogenic microorganisms' settlement into the hydrogel.

Another aim is to reduce the possibility of the body's tissue response to the implant by way of diminishing the amount of free radicals and monomers in the hydrogel.

These tasks were solved by the polyfunctional biocompatible hydrogel, which contained copolymer of cross-linked acrylamide with a cross-linking agent and water. According to the invention, the above-mentioned copolymer, being a cross-linking agent, contains a mixture of N,N'-methylene-bis-acrylamide, N,N'- ethylene - bis acrylamide and poviargolum with the following ratio of the components in mass%:
Acrylamide - 65,0 - 99,5,
N,N'-methylene-bis-acrylamide- 0,2 - 6,5,
N,N'-ethylene-bis-acrylamide- 0,2 - 34,0,
Poviargolum - 0,1 - 3,0.

As a cross-linking agent, the above-mentioned copolymer can also contain vinylpyrrolidone or ethylene-bis-(oxyethylenenitrilo)-tetraacetic acid or their mixture with the following ratio of the components in mass%:
Acrylamide - 65,0 - 99,4,
N,N'-methylene-bis-acrylamide- 0,2 - 6,5,
N,N'-ethylene-bis-acrylamide- 0,2 - 34,0,
Poviargolum - 0,1 - 3,0,
Vinylpyrrolidone or ethylene-bis-(oxyethylene-nitrilo)-tetraacetic acid), or their compounds - 0,1-2,5.

The above-mentioned biocompatible hydrogel contains bi-distilled apyrogenic water.

The biocompatible hydrogel has pH of 3,5 - 7,5.

The above-mentioned cross-linked copolymer constitutes from 2,0 to 15,0% of the whole mass of the hydrogel.

The biocompatible hydrogel contains the following ratio of the components in mass%:
Acrylamide - 1,3 - 15,
N,N'- methylene-bis-acrylamide0,004 - 0,975,
N,N'- ethylene-bis-acrylamide0,004 - 5,1,
Poviargolum - 0,002 - 0,45,
Water - up to 100.

The biocompatible hydrogel can also contain the following ratio of the components in mass%:
Acrylamide - 1,3 - 15,
N,N'-methylene-bis-acrylamide0,004 - 0,975,
N,N'-ethylene-bis-acrylamide0,004 - 5,1,
Poviargolum - 0,002 - 0,45,
Vinylpyrrolidone and/or ethylene-bis-(oxyethylene-nitrilo)-tetraacetic acid - 0,002 - 0,375, Water - up to 100.

The raised tasks are also solved by the proposed method of production of the polyfunctional biocompatible hydrogel by way of copolymerization of acrylamide with the cross-linking agent in an aqueous medium in the presence of peroxide initiator of polymerization at incubation of the reaction mixture in two stages. The first stage is conducted at the temperature of 20 - 90°C during 2 - 24 hours. In accordance with the invention, the cross-linking agent shall be the mixture of N,N'- methylene-bis-acrylamide, N,N'- ethylene-bis-acrylamide and poviargolum with the following ratio of the components in mass%:
Acrylamide - 1,3 - 15,
N,N'-methylene-bis-acrylamide 0,004 - 0,975,
N,N'-ethylene-bis-acrylamide 0,004 - 5,1,
Poviargolum - 0,002 - 0,45,
Water - up to 100,

The second stage of the reaction mixture's incubation is conducted at the temperature of 107 - 130 °C for not more than 2 hours.

The method can be used even when the cross-linking agent is represented by a compound of N,N'-methylene-bis-acrylamide, N,N'-ethylene-bis-acrylamide, poviargolum, vinylpyrrolidone and/or ethylene-bis-(oxyethylene-nitrilo)-tetraacetic acid with the following ratio of the components in mass%:
Acrylamide - 1,3 - 15,
N,N'-methylene-bis-acrylamide 0,004 - 0,975,
N,N'-ethylene-bis-acrylamide 0,004 - 5,1,
Poviargolum - 0,002 - 0,45,
Vinylpyrrolidone and/or ethylene-bis-(oxyethylene-nitrilo)-tetraacetic acid - 0,002-0,375, Water - up to 100.

After the first stage of the reaction compound's incubation, the hydrogel is washed in hot water of 70 - 110 °C for at least 3 hours. The mass ratio of the hydrogel and water must:be 1:8 - 10.

The initiator of polymerization must be represented by hydrogen peroxide and/or ammonium persulfate in the amount not more than 0,33 mass% of the total weight of the original components.

Bi-distilled apyrogenic water shall form the aqueous medium.

It is known that the substance in the form of the hydrogel on the basis of copolymer of acrylamide and cross-linking agents shall represent a three-dimensional network of cross-linked copolymer of acrylamide with cross-linking agents. In its cells an aqueous medium is hold and it contains a certain unestablished amount of polymerization's unconnected initiator as some unestablished amount of the polymerization's initiator got built directly into copolymer's structure (see Savitskaya M.N., Kholodova Y.D. "Polyacrylamide", Publishing House "Tekhnika", 1969, p.103) or washed out of the hydrogel during its washing.

At that biologically active characteristics of such hydrogel for the most part depend on the structure of the cellular polymer, which in its part depends on the conditions of its synthesis, i.e. quantitative and qualitative ratio of original reagents, including cross-linking agents and initiators of polymerization, which got built into the copolymer structure by chemical and hydrogenous connections (by groups NH, CH, COOH, NH2, CH2), as well as temperature conditions of polymerization.

The main point of the invention lies in the fact that inclusion of N,N'-ethylene-bis-acrylamide and poviargolum as cross-linking agents into the hydrogel, based on acrylamide and N,N'-methylene-bis-acrylamide as well as selection of the connections for copolymerization allowed to diminish the amount of unconnected amides, free NH2 radicals and unlimited double connections. It also became possible to increase the cross-linking rate at the expense of structural groups' formation (HC-NH-CH), (-CO-NH-CR-O-R), (-CO-NH-NH-CO-), (H-COR-NH-CR-O-R), (-CONH-R-NH-CO), where R- CH₃, CH₂, NH₂, C₂H₅, and increase in the amount of cross-links - N-N bonds.

It allows to reduce the tissue response of the organism to the implantation of the offered hydrogel, provide for the high stability of its form during implantation at the expense of reduction of its resorption and shrinkage rate in the patient's organism and to reduce the possibility of its settling with microorganisms, including the ones from the recipient's body, and of reproduction of these microorganisms.

### Brief Description of Illustrations.

In order to provide for a better understanding of the invention, below are the examples of particular production of the offered biocompatible hydrogel with references to the attached illustrations, where:
Fig. 1a represents infra-red absorption spectrum for the offered hydrogel;
Fig. 1b represents infra-red absorption spectrum for the hydrogel-prototype, manufactured in Russia under patent RU No. 2127129 under trademark "Formacryl";
Both infra-red spectra are executed in the area of 4000-500 cm-1 (axis "x" represents the length of the light wave (cm-1); axis "y" - the degree of light absorption T (in %);
Fig.2a represents chromatogram of the offered hydrogel's extract;
Fig.2b represents chromatogram of the extract of hydrogel "Formacryl".
Both chromatograms were received by way of highly effective fluid chromatography with ultra-violet detector with the wave's length of 240 nm and they are made on chromatographic paper, where the left column of figures represents the height of peaks,
   where peak 1 corresponds to the time of keeping of the dissolvent (acetonitril) on the column, peak 2 is the quantitative reflection of the amount of monomers in the hydrogel's extract, received with the help of the above-mentioned dissolvent. A unit (1 cm) of the height of peak 2 at the chromatogram corresponds to 0,057 mcg of monomers, contained in 1 g of polymer (0,057 ppm).

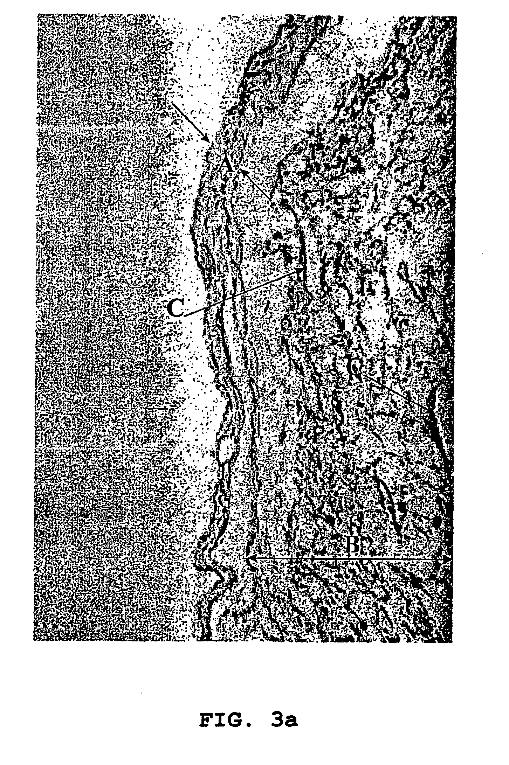

Fig.3a represents a photo of histological section of biopsy of rat's tissue, which was taken 30 days after subcutaneous injection of the offered hydrogel (colouring is made by hematoxylin-eosin, x200);

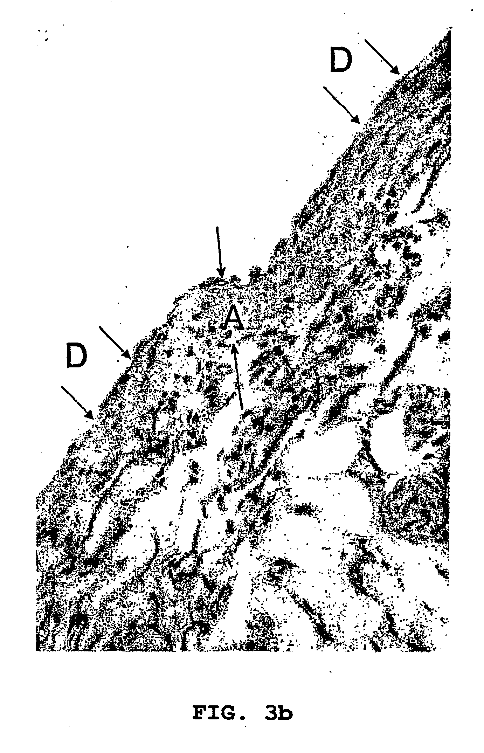

Fig.3b represents a photo of histological section of biopsy of rat's tissue, which was taken 30 days after subcutaneous injection of hydrogel "Formacryl" (colouring is made by hematoxylin-eosin, x200);

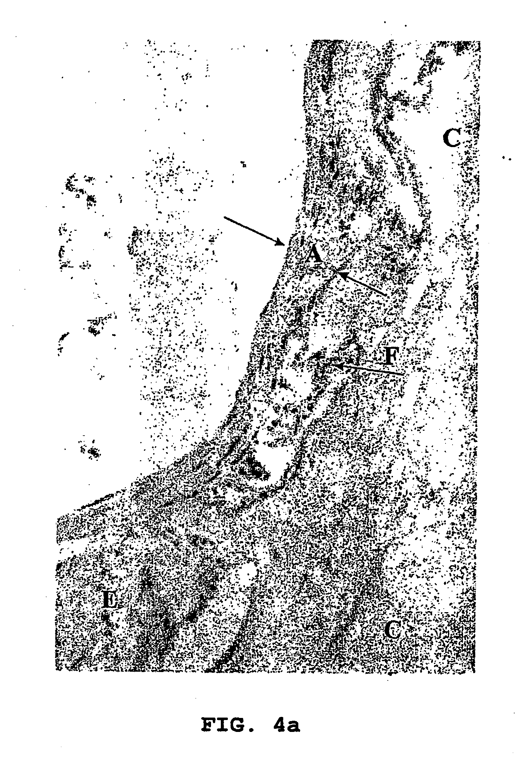

Fig. 4a represents a photo of histological section of biopsy of dog's tissue, which was taken 9 months after subcutaneous injection of the offered hydrogel (colouring is made by hematoxylin-eosin, x200);

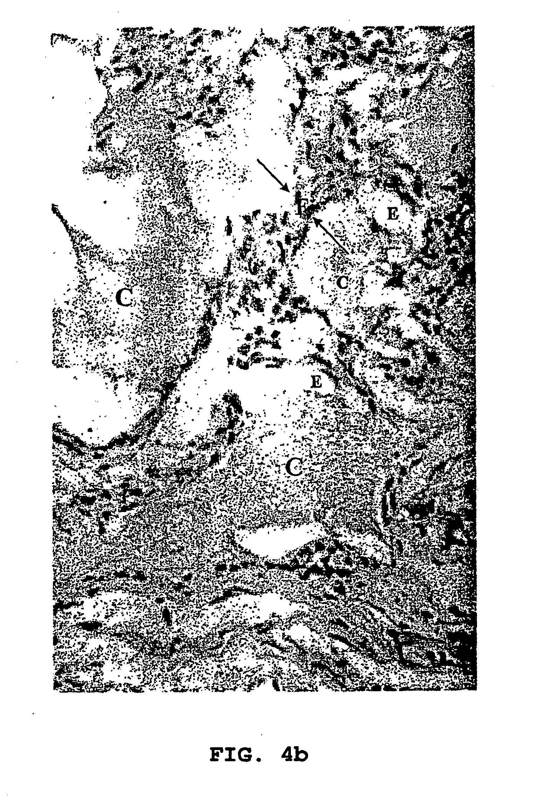

Fig.4b represents a photo of histological section of biopsy of dog's tissue, which was taken 9 months after subcutaneous injection of hydrogel "Formacryl" (colouring is made by hematoxylin-eosin, x400);

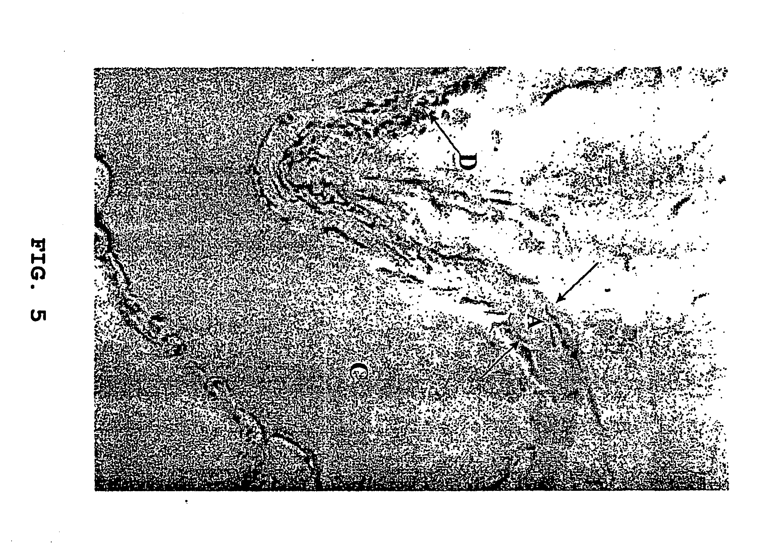

Fig.5a represents a photo of histological section of biopsy of human tissue, which was taken 12 months after subtonsils implantation of the offered hydrogel for mammoplasty (colouring is made by hematoxylin-eosin, x400), wherein
   A - a connective capsule,
   B - zone behind the capsule,
   C - a fragment of the hydrogel,
   D - macrophages on the surface of capsule A from the side of the implant,
   E - zone of the hydrogel's lysis,
   F - cords of the connective tissue.

### Versions of the Invention's realization.

In order to receive the offered biocompatible hydrogel we must take:
- Acrylamide: C₃H₅NO, molecular weight - 71.08, white crystalline powder without any smell; melting temperature 84,5 °C; made by Sigma (Catalogue «Reagents for Biochemistry and Research in the Area of Natural Sciences» SIGMA, 1999, p. 47, catalogue No. A8887);
- N,N'-methylene-bis-acrylamide: C₇H₁₀N₂O₂, molecular weight - 154,16, white crystalline powder without any smell; melting temperature 185 °C, made by Sigma (Catalogue «Reagents for Biochemistry and Research in the Area of Natural Sciences» SIGMA, 1999, p. 696, catalogue No.M7256);
- N,N'-ethylene-bis-acrylamide: C₈H₁₂N₂O₂, molecular weight - 168,2, made by Sigma (Catalogue «Reagents for Biochemistry and Research in the Area of Natural Sciences» SIGMA, 1999, p. 428, catalogue No. E2763);
- Poviargolum-argentiferous detergent, powder, No.97/167/7 from Institute of High-Molecular Compounds of Russian Academy of Sciences (Russia), F.G.: 11.2 (Register of Drugs, Used in Russia - Aptekar, 2001, p.1067), which is a superfine metal silver, stabilized by poly-N-vinylpyrrolidone-2 (see WWW: http://home.comset.net/poviarg/; RU 2088234, published on 27.08.97);
- ethylenebis(oxyethylenenitrilo)-tetraacetic acid: [-CH₂OCH₂CH₂N(CH₂CO₂H₂)₂]₂, molecular weight 380,35, melting temperature 249 °C, made by Aldrich (Catalog handbook of Fine Chemicals Aldrich, 1994-1995, p.664, catalogue No. 23,453-2);
- 1-vinyl-2-pyrrolidone: C₆H₉NO, molecular weight 111,4; melting temperature 93 °C; white powder; made by Fluka (Catalogue Fluka Chemika-Biochemika, Switzerland, sFr, 1993/94, p.1384, catalogue No. 95060);
- Ammonium persulfate: (NH₄)₂S₂O₈ - molecular weight 228.19; colourless flat crystals; destruction temperature 120 °C; made by Sigma (Catalogue «Reagents for Biochemistry and Research in the Area of Natural Sciences» SIGMA, 1999, p.117);
- Hydrogen peroxide: H₂O₂- molecular weight 34,0; colourless fluid, density at 0 °C -1,465; melting temperature -0,89 °C; made by Sigma (Catalogue «Reagents for Biochemistry and Research in the Area of Natural Sciences» SIGMA, 1999, p. 556, catalogue No. H6520);

All above-mentioned monomers shall be suitable for biological purposes and shall not require additional cleaning.

Water shall be bi-distilled and apyrogenic (pH = 5,6).

The method must be conducted the following way:
In order to prepare the reaction compound we take bi-distilled apyrogenic water with pH of 5,6.

We prepare an aqueous solution of acrylamide and cross-linking agents, for example, the compound of N,N'-methylene-bis-acrylamide, N,N'-ethylene-bis-acrylamide and poviargolum (taken in the given mass ratio within the following limits: 65,0 - 99,5 : 0,2 - 6,5 : 0,2 - 34,0 : 0,1 - 3,0), or a compound of N,N'-methylene-bis-acrylamide, N,N'-ethylene-bis-acrylamide, poviargolum, vinylpyrrolidone or ethylenebis(oxyethylenenitrilo)-tetraacetic acid, or sums of the latter two (taken in the given mass ratio within the following limits: 65,0 - 99,4 : 0,2 - 6,5 : 0,2 - 34,0 : 0,1 - 3,0 : 0,1-2,5), at that the total mass of original monomers in the solution makes up 2,0-15,0 %. (By modifying the amount of the original monomers in the solution, we get the hydrogel of different density and flexibility).

Into the received solution we introduce polymerization initiators hydrogen peroxide in the amount of 0,1 - 0,3 mass% or ammonium persulfate in the amount of 0,0006 - 0,03 mass%, or their compound in any correlation and amount, not exceeding the sum of their maximum values. By modifying the amount of hydrogen peroxide and ammonium persulfate, we receive the substance of the desired pH within the range of 3,5 - 7,5.

The finished reaction compound is filtered through the bactericidal polymer filters, for example of brand F8273 with the pores' size of 0,45 mm CA/CN, made by Sigma (USA), and placed for incubation at the temperature of 20 - 90 °C for 2 -24 hours. After the incubation, the hydrogel, which looks like a gel, is washed by hot water. For that purpose the gel is placed into a reservoir with water of 90 - 100 °C with the ratio of the gel's and water amount of 1 : 8 - 10 for 4 - 6 hours. Then the second stage of the incubation is carried out at the temperature of 107 - 130 °C for 1,0 - 1,5 hour.

The received gel is packed in the necessary amount into bottles or syringes and sterilized by autoclaving (at the temperature of 120°C, p = 1,2 atm) for 20 minutes.

We carried out physical and chemical, medical and toxicological examinations of the offered gel's samples, including those received in the below-mentioned Examples 1 - 5, in accordance with ISO 10993 "Evaluation of Biological Effect of Medical Devices", "Methodical Directions on Sanitary and Hygienic Evaluation of Rubber and Latex Devices, Meant for Medical Purposes" (Ministry of Public Health of USSR, Moscow, 1988) and Methodic recommendations "Admissible amount of migration of chemical agents, that are exuded from polymer and other materials in contact with food hydrogels and methods of their determination" Sanitary Rules and Standards 42-122-42-40-86.

The determination of monomers' content for acrylamide, N,N'-methylene-bis-acrylamide, N,N'-ethylene-bis-acrylamide and ethylenebis(oxyethylenenitrilo)-tetraacetic acid were carried out in accordance with the methods, described in the work of V.V. Kuznetsov et al. «Determination of Acrylamide in Polyacrylamidic gels"// The 52-nd Pittsburgh Conference on Analytical Chemistry and Applied Spectroscopy. - New Orleany, LA, 2001, Abstract Book, No. 1648.

These researches revealed that the offered hydrogel has the following physical and chemical characteristics:
- Appearance - gel
- Colour - from colourless to semitransparent dark brown, opalescent;
- Refractive index - 1,328-1,360;
- Density - 1,0 - 1,2g/cm3;
- pH - 3,5 - 7,5;
- Monomers' content - up to 0,4 ppm;
- Bromation rate - not more than 1,0 (mg of bromine for 1 l).

Sanitary and chemical examination revealed the following:
- Migration of metals - Cu, Fe, Ni, Zn, Al, Ti, Ag from the hydrogel into the aqueous extract, determined by an atomic absorptive method, was not found within the limits of the method's perceptibility (0,02; 0,05; 0,05; 0,02; 0,005; 0,04 mg/l correspondingly), which is a considerably lower figure than those, admitted for drinkable water;
- Migration of sodium constituted not more than 0,12 mg/l at the admissible level of 200 mg/l for drinkable water;
   Toxicological examination showed that aqueous extracts from the hydrogel did not reveal a hemolytic effect in the "in vitro" experiments with isolated erythrocytes of rabbits. The hemolysis was set at 0,04% at the admissible rate of 2%.

Zone of growth inhibition of Staphylococcus aureus around the disks, cut out of the samples, determined as described in the methodic guide "Method for Determination of Microorganisms' Antibiotic Perceptibility by Disk Diffusion Technique", Moscow, Ministry of Public Health, 1984, constituted 1,5 - 3 mm, depending on the density of the sample (polyacrylamide content).

In the acute experiment on white mice at the parenteral introduction of the hydrogel's samples in the amount of 50,0 ml for 1 kg of the weight, there were neither loss of animals nor clinical signs of intoxication: general condition of the mice under the experiment, their behaviour, eating, state of hair had no difference from those under control.

Autopsy of the mice under experiment showed that the tissues at the place of hydrogel's injection, regional lymph nodes, internals (liver, kidneys, spleen) were within the boundaries of physiologic norms and control.

We have not found any statistically reliable differences in dynamics of body weight, clinical and biochemical blood indices, coefficients of internals of the animals under experiment in comparison with the control group at the subcutaneous implantation of the gel for 2,5 months. We have not found any sensibilizing effect of the hydrogel at conducting an immunologic diagnostic reaction of mast cells' degranulation reaction.

Microkernel test on the marrow showed no mutagenic effect of the hydrogel's action. Histological examination of area, where the hydrogel was implanted, and internals (liver, kidneys, spleen, testicles) showed a slight tissue response to the hydrogel only in the first days after the implantation. There were no dystrophic and necrotic changes in the organs.

Below there are the specific examples of how the offered biocompatible hydrogel is produced and used for soft-tissue plasty.

### Example 1.

In order to receive the hydrogel we took 384 ml of bi-distilled apyrogenic water with pH 5,6 and dissolved in it 13g of acrylamide, 5,5 g of N,N'-methylene-bis-acrylamide, 2,3 g of N,N'-ethylene-bis-acrylamide and 0,2 g of poviargolum, suitable for biological purposes. Then the initial solution was combined with 0,04g of ammonium persulfate and 2ml of 30% hydrogen peroxide. The received compound was filtered through a bactericidal polymeric filter of F8273 brand with the pores of 0,45mm CA/CN, made by Sigma (USA), and put into the reservoir, which was placed for incubation on a water bath at the temperature of 30°C for 22 hours. Then the hydrogel in the form of a gel was washed in hot water with the ratio of water and the gel - 10:1 at the temperature of 90°C for 4 hours and again incubated for 1 hour at the temperature of 125°C.

The received hydrogel was sterilized by autoclaving (at the temperature of 120°C and pressure 1,2 atm.) for 20 minutes.

The received gel contains 96 mass% of the water phase and 4 mass% of copolymer, in which acrylamide makes up 81,25 mass%, N,N'-methylene-bis-acrylamide - 3,125 mass%, N,N'-ethylene-bis-acrylamide - 14,375 mass% and poviargolum - 1,25 mass%. It has pH =5,4.

We examined the following characteristics of the hydrogel:

Refraction index (by the methods, described in "Practical Work on Physical Chemistry", Moscow, 1974, pp 86 - 97); - pH (by the methods, described in the book "Methodical Directions on Sanitary and Hygienic Evaluation of Rubber and Latex Devices, Meant for Medical Purposes", Moscow, 1988, pp 18 - 19); - bromation level (by the methods, described in "Collected Guide Materials On Toxicological Examination of Polymeric Materials and Devices for Medical Purposes, Made on Their Bases", Moscow, Ministry of Public Health of USSR, 1987, pp.27 - 29);-monomers' content - by the methods, we developed for determination of monomers' content in hydrogenous polymers: V.V. Kuznetsov et al. «Determination of Acrylamide in Polyacrylamidic Gels"// The 52-nd Pittsburgh Conference on Analytical Chemistry and Applied Spectroscopy. - New Orleans, LA, 2001, Abstract Book, No.1648.

The received sample of the gel had the following physical and chemical characteristics:
Appearance - colourless, semitransparent, opalescent gel;
Refraction index - 1,348;
pH - 5,4;
Density - 1,0g/cm3;
Monomers' content - 0,1 ppm;
Bromation level - 0,1(mg of bromine for 11).

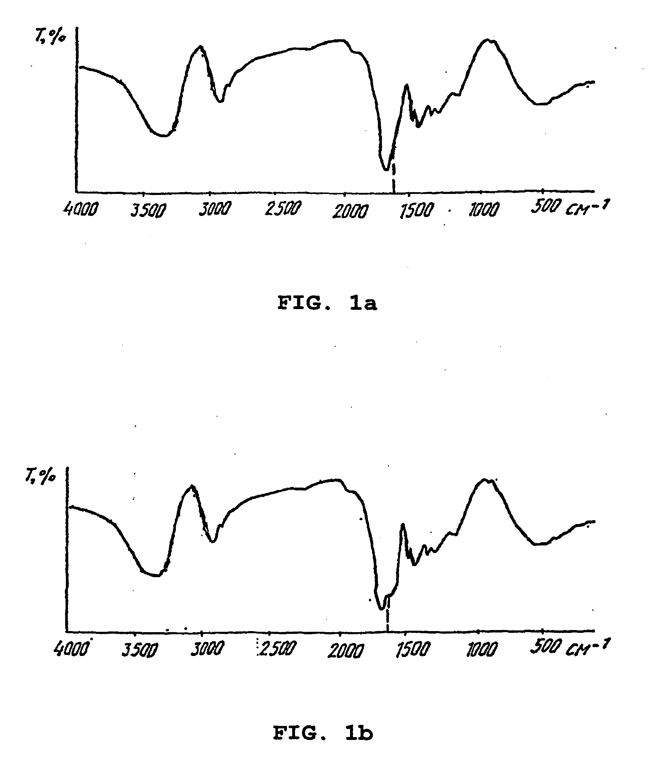

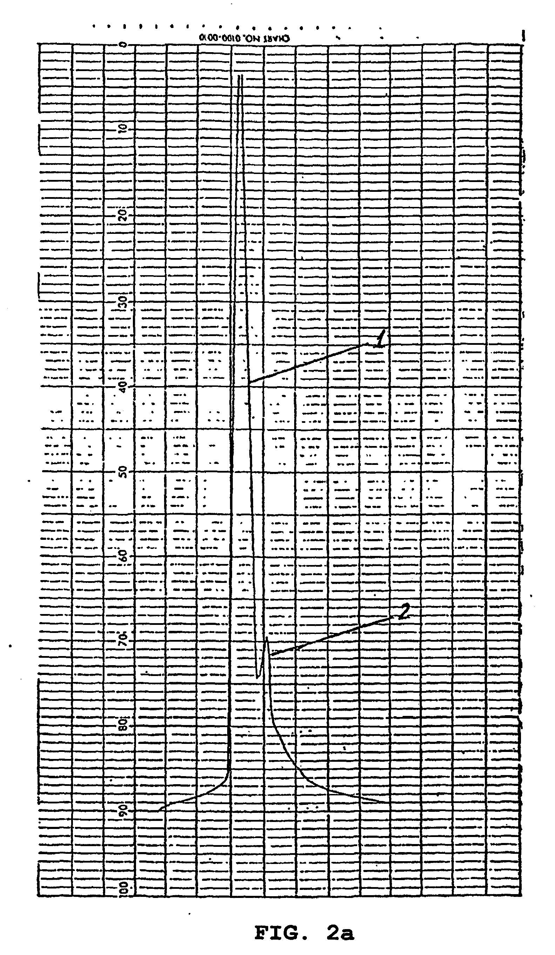

We received infra-red spectrum and chromatogram of the hydrogel's extract, represented correspondingly on Fig. 1a and Fig. 2a.

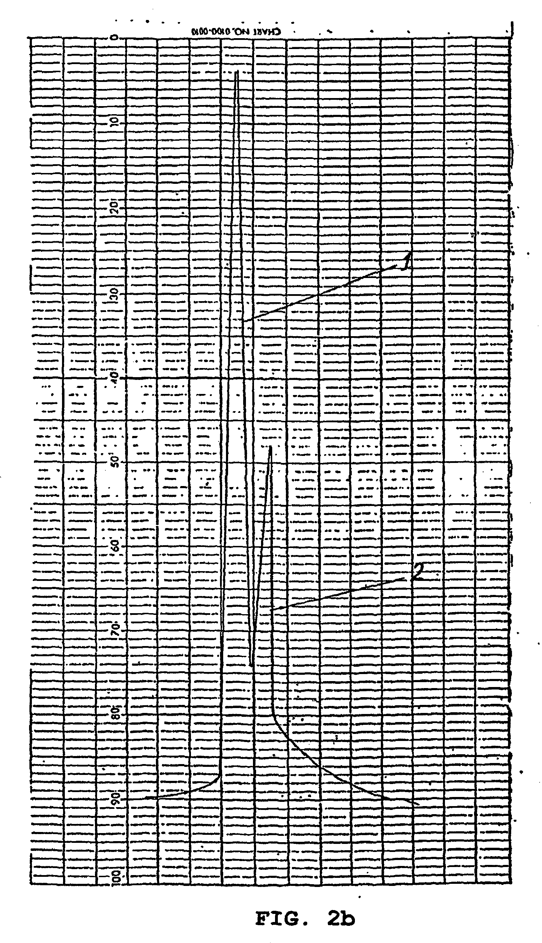

For comparison Fig. 1b and Fig. 2b represent infra-red spectrum and chromatogram of an extract of the well-known hydrogel-prototype, manufactured in Russia under patent RU No.2127129 under the trademark "Formacryl", which contains 96 mass% of the water phase and 4 mass% of the copolymer, composed of 96 mass% of acrylamide and 4 mass% of N,N'-methylene-bis-acrylamide. It has pH =5,4, bromation level 0,27 (mg of bromine for 11), and which was received at the incubation of the initial compound in the presence of hydrogen peroxide and ammonium persulfate in the total amount of 0,3 mass% at the temperature of 60°C for 12 hours and then at the temperature of 100°C for 2 more hours.

As it is evident from the spectrum, represented on Fig. 1a, there are no zones of 1620 cm-1, responsible for the deformation fluctuations of NH₂ radicals, and zones of 3200 cm-1 and 3600 cm-1, responsible for the valency fluctuations of these radicals. It is indicative of the fact that free NH₂ radicals constitute not more than 1% of the total number of functional groups in the polymer's structure.

As it is evident from spectrum, represented on Fig. 1b, there is an insignificant zone of 1620 cm-1, which is indicative of the fact that there a bit more than 1% of NH₂ radicals.

As indicates the chromatogram on Fig 2a, peak2, which corresponds to the total amount of monomers of acrylamide, N,N'-methylene-bis-acrylamide, N,N'-ethylene-bis-acrylamide, does not exceed 5,1 cm, that corresponds to the monomers' content in the amount of 0,29 ppm.

As is obvious from the chromatogram, represented on Fig. 2b, the content of monomers of acrylamide and N,N'-methylene-bis-acrylamide in Formacryl constitutes 0,6ppm, the height of the peak is 10,7 cm.

We also studied the bacteriostatic features of the received sample of the hydrogel. We used disk-diffusive method with the application of Staphylococcus aureus strain.

It was established that the zone without cocci's growth around the sample constituted 1,5 ± 0,1 mm, while there was no zone without bacterial growth around the control sample, represented by hydrogel "Formacryl" of the same density. Moreover, we observed the growth of bacteria directly under the disk of hydrogel "Formacryl".
We examined the shrinkage rate of the offered hydrogel in comparison with the similar characteristics of hydrogel "Formacryl". For that we made a subcutaneous implantation of 30 ml of samples of the offered hydrogel and hydrogel "Formacryl", to dogs, in particular, with the subsequent taking the samples out and testing of dry residue and water phase of the hydrogel in one, three and six months after the implantation. It was established that even when the offered implant stayed in the animal's organism for 6 months, the loss of water phase constituted not more than 5% of its original content in the implant, while the loss of the water phase in the implanted samples of hydrogel "Formacryl" constituted about 10% in the similar conditions. The amount of dry residue in both cases was practically unchanged.

We examined the tissue response to the implantation of the received sample of the hydrogel in experimental-morphological and clinical-morphological studies.

The study was carried out on 160 male rats of August line, weighing 200g, and 10 dogs.

The rats received a 1 ml injection of the hydrogel subcutaneously.

The terms of the morphological study constituted 3, 7, 14, 30, 60 and 90 days.

The long-term implantation (6 - 12 months) was examined on dogs, that were injected 15 ml of the hydrogel subcutaneously.

In the clinic the morphological study of tissues after the implantation of the same hydrogel to 5 patients was carried out: 1 month after the implantation under the skin in the facial area, 1,5 and 3,5 months after intramuscular implantation into crus, and 6 and 12 months after the implantation, meant for increasing mammoplasty by way of filling the fibrous capsule's cavity with the gel after silicon prostheses were taken out.

In order to carry out the histological study, the tissue blocks were fixed in 96° ethyl alcohol and neutral formalin and covered by paraffin. The cuts were coloured by hematoxylin-eosin, picrofuxin by Van-Gizon, silvering by Gommori to study the fibrous components, blue toluidine for soar glycosaminoglycans, studied PAS-reaction for glycogen and glycoprotein as well as Brachet reaction for RNA.

Some results of the histological studies are represented on Fig. 3a, 4a, 5.

Morphologic study of the organism's tissue response to the implantation of the received sample of the offered hydrogel, conducted on rats, shows the minimal response. In the early days (3 - 7 days after the implantation) the response is limited to a slight lympho-macrophage infiltration with single neutrophils and feeble tissue edema. It is indicative of a minimal inflammatory response. On the third day we observe the proliferation of fibroblasts in the narrow zone around the implant, and on the seventh day there has been formed a very thin connective capsule, which consists of fibroblasts and thin collagen fibers. From inside the capsule is covered with a layer of macrophages, bordering the implant. In 14 days the capsule is more clearly defined, but it is still thin and friable. Inside the capsule, as well as between it and the cellular tissue (behind the capsule) we see small fragments of the hydrogel, surrounded by macrophages and single giant multinucleous cells. 30 days after the implantation the capsule A (Fig. 3a) remains very thin. It consists of the mature connective tissue, the amount of fibroblasts in which is diminishing, and in the remaining cells the content of RNA is diminishing. Behind the capsule, in zone B there are still fragments C of the hydrogel, which are resorbed by macrophages. The inner surface of the capsule is partially covered by macrophages. In the future terms (60 and 90 days) the structure of the capsule remains unchanged, inside the gel there come some cords of fibroblasts from the capsule.

For comparison, Fig. 3b represents the photo of the histological cut of the rat tissue biopsy, taken 30 days after the subcutaneous injection of the hydrogel-prototype ("Formacryl"), which consists of 96 mass% of the water phase and 4 mass% of the copolymer, 96 mass% of which is acrylamide, 4,0 mass% is N,N' - methylene - bis - acrylamide. It has pH = 5,4, bromation level of 0,27 (mg of bromine for 1 l) and was received at the incubation of the initial compound in the presence of hydrogen peroxide and ammonium persulfate in the total amount of 0,3 mass% at the temperature of 60°C for 12 hours, and then at the temperature of 100°C for 2 more hours. As it is seen from the photo on Fig. 3b, the connective capsule A around the implant is much thicker than at the implantation of the offered substance. Inside the capsule in its inner surface and in the capsuled zone there is an increased number of macrophages D, giant multinucleous cells and neutrophils, which is indicative of a slight inflammatory process.

The tissue response in the later terms (6, 9 and 12 months) was studied on a subcutaneous implantation of the hydrogel to dogs. As it is seen from Fig. 4a, 9 months after the implantation the implanted hydrogel C retains its homogeneous structure for the most part. Near a very thin and dense connective capsule A there is a narrow zone E of the hydrogel's enlightment (lysis), which is resorbed by macrophages and germinated by cords F of fibroblasts. There is no deep infiltration of cells into the hydrogel. It is the cause of its long-term stability. There are no lime deposits in the capsule or in the hydrogel. There are dystrophic changes of the cells in the tissue around the implant, which could be indicative of the toxicological influence of the hydrogel.

For comparison, Fig. 4b represents a photo of the histological cut of the dog's tissue, taken 9 months after the subcutaneous implantation of the sample (the same as for Fig. 3b) of the hydrogel-prototype (colouring by hematoxylin-eosin, x400).

As it is seen from the photo gel C is germinating with cords F of the connective tissue, which contains fibroblasts, macrophages, leukocytes and single neutrophilic leukocytes. There is a partial lysis E of the gel and its resorption.

Clinical and morphological studies, conducted 1 month after the injection of 90 ml of the sample of the offered hydrogel for dermotension of the skin and fat part of the face with the purpose of the subsequent cicatrices plasty, showed that on the border between the hydrogel and tissues there is forming a very thin and friable connective tissue, made of only several layers of collagen fibres and fibroblasts. The cellular lympho-macrophagal infiltration is minimal. In some zones outside the capsule there can be seen tissue vacuoles, remained in place of the resorbed gel. There is a slight macrophagal and gigantocytous response.

The similar results were received in two cases of study of contour plasty of crus' soft tissues by way of injection of the hydrogel, received by the offered method. 1,5 and 3,5 months after the implantation the gel retained homogenous for the most part and was germinating by the connective tissue only around the capsule. Fig. 5 represents the results of the morphological study of biopsy, taken 12 months after the injection of the sample of the offered hydrogel in the amount of 200 ml in place of taken-out silicon prosthesis in the cavity of the remained fibrous capsule.

As it can be seen on Fig. 5, the tissue response to the gel is slightly expressed. The "old" fibrous capsule is subject to a reverse development nearly everywhere. Implant C is surrounded by a thin connective capsule A without inner miofibroblastic layer, which is present in the capsules around the silicon prosthesis. Somewhere in the "new" capsule there are not numerous macrophages D and lymphocytes without any inflammatory neutrophilic reaction. Vessels of the capsule are not numerous, there are no dystrophic changes and deposits of calcium salts.

Near the capsule A there is a rather shallow germination of thin cords of connective tissues (fibroblasts, macrophages and thin immature collagen fibres) into the hydrogel. Some macrophages have a large foamy cytoplasm (active phagocytosis). Cords of the connective tissue divide the gel near the capsule into the fragments.

Thus, the results of the histological studies, received in a long dynamics on animals, as well as on bioptic clinical material, are indicative of the high biocompatibility of the offered hydrogel.

In the early terms after the injection of the samples of the offered hydrogel into the organism, we observed a very feeble and quickly disappearing inflammatory response, fibroblastic response was slow and feeble, the capsule was formed late and remained thin during the whole period of observation.

It is characteristic that there was no deep invasion of macrophages and microphages into the hydrogel. It confirms the stability of the hydrogel towards the resorption in the organism. At that the hydrogel does not diminish the functional activity of the cells and does not lead to their dystrophy, which is indicative of the absence of toxic substances' migration from it into the tissue. There is also no calcification of the hydrogel and surrounding tissues.

The received hydrogel was injected to patient L-kaya, 55 years old, instead of the hydrogel which was used for initial mammoplasty 5 years earlier and which led to the inflammatory process in both mammary glands. In the postoperative period the patient was observed for 8 months with monthly examination. There was no recurrence of the inflammatory process. We received a positive result: mammary glands acquired the form and size, corresponding to the patient's constitution, and elasticity, characteristic of the tissue of a healthy mammary gland.

### Example 2.

In order to receive the hydrogel we took 870 ml of bi-distilled apyrogenic water with pH 5,6 and dissolved in it 81,0g of acrylamide, 11,5g of N,N'-methylene-bis-acrylamide, 32,9g of N,N'-ethylene-bis-acrylamide and 4,6g of poviargolum. Then the initial solution was combined with 8ml of hydrogen peroxide in order to initiate the polymerization. The received compound was filtered as described in Example 1, placed for incubation at the temperature of 80°C for 2 hours. Then the gel was washed in 10 liters of hot water at the temperature of 80°C for 5,5 hours and again incubated for 1,5 hours at the temperature of 125°C.

The received hydrogel was sterilized as described in Example 1.

The hydrogel had the following physical and chemical characteristics, which were determined in accordance with the methods, specified in Example 1:
Appearance - semitransparent, light yellow gel;
Refraction index - 1,336;
pH - 4,0;
Density - 1,0g/cm3;
Content of monomers of acrylamide and bisacrylamide - up to 0,4 ppm;
Bromation level - 0,9(mg of bromine for 11);
Bacteriostatic features
Zone of bacteria's growth suppression - 2,5 mm.

The received hydrogel was used for gastronemius muscle plasty. The hydrogel in the amount of 50ml was injected into the gastrocnemius muscle of patient I., 42 years old, in order to eliminate the defect of the trauma she had received.

In the postoperative period the patient was observed for 6 months. There were no inflammatory reactions or edema. The gel's migration was absent. The cosmetic effect was achieved.

### Example 3.

In order to receive the hydrogel we took 972 ml of bi-distilled apyrogenic water with pH 5,6 and dissolved in it 22,5g of acrylamide, 0,375g of N,N'-methylene-bis-acrylamide, 5g of N,N'-ethylene-bis-acrylamide and 0,06g of poviargolum and 0,065g of ethylene-bis-(oxyethylenenitrilo)-tetraacetic acid. Then the initial solution was combined with 2,5g of ammonium persulfate. The received compound was filtered as described in Example 1, and placed for incubation at the temperature of 80°C for 2 hours. Then the gel was washed in 8 liters of hot water at the temperature of 100°C for 5,5 hours and again incubated for 1,5 hours at the temperature of 125°C.

The received hydrogel was sterilized as described in Example 1.

The hydrogel had the following physical and chemical characteristics, which were determined in accordance with the methods, specified in Example 1:
Appearance - colourless gel;
Refraction index - 1,334;
pH - 6,8;
Density - 1,0g/cm3;
Content of monomers of acrylamide - absent;
Content of the other monomers - 0,02 ppm;
Bromation level - 0,15(mg of bromine for 1l);
Bacteriostatic features
Zone of bacteria's growth suppression - 1,5 mm

We conducted a comparative study of the shrinkage rate of the samples of the offered hydrogel and its prototype "Formacryl", which contain the same amount of polyacrylamide - 2,8 mass%.

For that the same amount (30 ml) of the hydrogel's samples were injected subcutaneously to dogs and taken out 3 and 6 months after that.

The determination of dry residue and water phase in the taken-out implants showed that
- 4 months after the implantation Formacryl lost 20% of water, and 6 months after the implantation it lost 30% of water;
- 4 months after the implantation the offered hydrogel, which contained the compound of N,N'-methylene-bis-acrylamide, N,N'-ethylene-bis-acrylamide and poviargolum as a cross-linking agent, lost 10% of water, and 6 months after the implantation it lost 15% of water;
- 4 months after the implantation the offered hydrogel, which contained the compound of N,N'-methylene-bis-acrylamide, N,N'-ethylene-bis-acrylamide, poviargolum and ethylene-bis-(oxyethylenenitrilo)-tetraacetic acid, as a cross-linking agent, lost 5% of water, and 6 months after the implantation it lost 9% of water.

Thus, the introduction of ethylene-bis-(oxyethylene-nitrilo)-tetraacetic acid (or vinylpyrrolydone or the compound of these two substances) provides for the diminishing of the implant's shrinkage rate, which is more characteristic of the hydrogels with the low content of dry residue.

The hydrogel with a small content of dry residue, up to 3%, can be easily injected through thin needles and can be used for the plasty of facial soft tissues. However, without the introduction of ethylene-bis-(oxyethylene-nitrilo)-tetraacetic acid into the polymer's content the hydrogel can be shrinked for up to 30%. Addition of ethylene-bis-(oxyethylenenitrilo)-tetraacetic acid allows to receive a gel, which is, on the one hand, is easy to inject through a thin needle, and, on the other hand, has a shrinkage level up to 10%, characteristic of a hydrogel with a greater amount of dry residue.

The received hydrogel was used for the plasty of subcutaneous cellular tissue at the elimination of facial wrinkles. Hydrogel in the amount of 1 ml was injected to patient S., 47 years old. In the postoperative period the patient was observed for 12 months with regular examinations every three months. There were no inflammatory or allergic reactions. The desired cosmetic effect was achieved.

### Example 4.

In order to receive the hydrogel we took 965 ml of bi-distilled apyrogenic water with pH 5,6 and dissolved in it 28,7g of acrylamide, 2,08g of N,N'-methylene-bis-acrylamide, 3,5g of N,N'-ethylene-bis-acrylamide and 0,1g of poviargolum and 0,82g of vinylpyrrolidone. The received compound was filtered as described in Example 1, and placed for incubation at the temperature of 60°C for 12 hours. Then the gel was washed in 10 liters of hot water at the temperature of 100°C for 4,5 hours and again incubated for 1,5 hours at the temperature of 120°C.

The received hydrogel was sterilized by autoclaving as described in Example 1.

The substance had the following physical and chemical characteristics:
Appearance - colourless, semitransparent, opalescent gel;
Refraction index - 1,352;
pH - 5,2;
Density - 1,0 g/cm3;
Content of monomers - 0,04 ppm;
Bromation level - 0,05(mg of bromine for 1l);
Bacteriostatic features: zone of bacteria's growth suppression - 2,0 mm.

The received hydrogel was used for elimination of nasolabial folds. The hydrogel in the amount of 2g was injected to patient K., 27 years old. In the postoperative period the patient was observed for 12 months with the regular examinations every three months. There was no inflammatory reaction or edema. The desired cosmetic effect was achieved.

Addition of vinylpyrrolidone into the compound of cross-linking agents also allows to receive a hydrogel, which is easy to inject through a thin needle, as a gel with polyacrylamide's content of up to 4 mass% and has a shrinkage rate of up to 10%, which is characteristic of the gel with a greater amount of dry residue.

### Example 5.

In order to receive the hydrogel we took 375 ml of bi-distilled apyrogenic water with pH 5,6 and dissolved in it 23,75g of acrylamide, 1,075g of N,N'-methylene-bis-acrylamide, 0,145g of N,N'-ethylene-bis-acrylamide and 0,03g of poviargolum. Then the initial solution was combined with 0,02g of ammonium persulfate and 1ml of 30% hydrogen peroxide. The received compound was filtered as described in Example 1, and placed for incubation at the temperature of 50°C for 16 hours. Then the gel was washed in 3,5 liters of hot water at the temperature of 100°C for 4,0 hours and again incubated for 1 hour at the temperature of 130°C.

The received hydrogel was sterilized by autoclaving as described in Example 1.

The substance had the following physical and chemical characteristics:
Appearance - colourless gel;
Refraction index - 1,348;
pH - 4,8;
Density - 1,0g/cm3;
Content of monomers - 0,03 ppm;
Bromation level - 0,12(mg of bromine for 11);
Bacteriostatic features: zone of bacteria's growth suppression - 2,0 mm.

The received hydrogel was injected to patient L-ovaya, 36 years old, instead of a silicon prosthesis, which had been applied 3 years earlier for initial mammoplasty, which caused the fibrosis of both mammary glands 7 months after the operation. We conducted the operation to take out the silicon prosthesis with open capsulotomia and deferred injection of 180g of the received hydrogel into each gland. 3 months after we injected 100g more of the same hydrogel. In the postoperative period the patient was observed for 7 months with regular examinations every two months. There is no recurrence of the fibrosis. As a result of the operation, the mammary glands acquired the form and size, corresponding to the patient's constitution, and elasticity, characteristic for the tissue of a healthy mammary gland. The desired cosmetic effect was achieved.

### Industrial Applicability.

Thus, the above-mentioned examples confirm that the offered biocompatible hydrogel can be received with the help of the offered method.

Besides, the offered hydrogel, provokes practically no tissue response, no sensibilization of the organism, no dystrophic and necrotic changes. It can be used for endoprosthesis replacement and contour plasty of soft tissues as well as for endoprosthesis replacement of joints and as a synovial medium in joints and joints' prosthesis.

Compared to the well-known hydrogel-prototype (hydrogel "Formacryl"), the offered hydrogel has a lower tissue response of the organism to the implantation, a higher stability of the form during the implantation and a lower possibility of settling and germination of a pathogenic microflora in it.

The offered hydrogel can be used as a filling stuff for endoprosthesis, which have a capsule and a filling stuff, as well as a depot for drugs during long-term pharmacotherapy, for example, during treatment of tumors and abscesses, as well as a carrier for cultivation of human and animal's cells with a subsequent implantation of the hydrogel, containing the above-mentioned cells, into the organisms of the mammals.

The introduction of additional cross-linking agents, such as 1-vinyl-2-pirrolydone and/or ethylene-bis-(oxyethylenenitrilo)-tetraacetic acid, into the offered hydrogel, provides for diminishing of its shrinkage without changing of other physical and mechanical characteristics.

## Claims

1. A polyfunctional biocompatible hydrogel, containing a cross-linked copolymer of acrylamide with a cross-linking agent and water, **characterized in that** this copolymer include a compound of N,N'-methylene-bis-acrylamide, N,N'-ethylene-bis-acrylamide and poviargolum as a cross-linking agent.

2. The polyfunctional biocompatible hydrogel, according to claim 1, **characterized in that** the above-mentioned cross-linked copolymer of acrylamide with a cross-linking agent contains the following ratio of the components, in mass%:
Acrylamide - 65,0 - 99,5,
N,N'-methylene-bis-acrylamide - 0,2 - 6,5,
N,N'-ethylene-bis-acrylamide - 0,2 - 34,0,
poviargolum - 0,1 - 3,0.

3. The polyfunctional biocompatible hydrogel, according to claim 1, **characterized in that** it contains the following ratio of the components, in mass%:
Acrylamide - 1,3 - 15,
N,N'-methylene-bis-acrylamide 0,004 - 0,975,
N,N'-ethylene-bis-acrylamide 0,004 - 5,1,
poviargolum - 0,002 - 0,45,
water - up to 100.

4. The polyfunctional biocompatible hydrogel, according to claim 1, **characterized in that** the above-mentioned cross-linked copolymer contains vinylpyrrolydone and/or ethylene-bis-(oxyethylenenitrilo)-tetraacetic acid as a cross-linking agent.

5. The polyfunctional biocompatible hydrogel, according to claim 4, **characterized in that** the above-mentioned cross-linked copolymer of acrylamide with a cross-linking agent contains the following ratio of the components, in mass%:
Acrylamide - 65,0 - 99,4,
N,N'-methylene-bis-acrylamide - 0,2 - 6,5,
N,N'-ethylene-bis-acrylamide - 0,2 - 34,0,
poviargolum - 0,1-3,0,
vinylpyrrolydone and/or ethylene-bis-(oxyethylene-nitrilo)-tetraacetic acid - 0,1-2,5.

6. The polyfunctional biocompatible hydrogel, according to claim 4, **characterized in that** it contains the following ratio of the components, in mass%:
Acrylamide - 1,3 - 15,
N,N'-methylene-bis-acrylamide 0,004 - 0,975,
N,N'-ethylene-bis-acrylamide 0,004 - 5,1,
poviargolum - 0,002 - 0,45,
vinylpyrrolydone and/or ethylene-bis-(oxyethylene-nitrilo)-tetraacetic acid - 0,002 - 0,375,
water - up to 100.

7. The polyfunctional biocompatible hydrogel, according to claim 1, **characterized in that** the mass of the above-mentioned cross-linked copolymer makes up from 2,0 to 15,0 mass% of the total mass of the above-mentioned biocompatible hydrogel.

8. The polyfunctional biocompatible hydrogel, according to anyone of the claims 1 - 6, **characterized in that** it contains bi-distilled apyrogenic water.

9. The polyfunctional biocompatible hydrogel, according to anyone of the claims 1 - 7, **characterized in that** it has pH 3,5 - 7,5.

10. A method of producing the polyfunctional biocompatible hydrogel by way of copolymerization of acrylamide with a cross-linking agent in aqueous medium in the presence of a peroxide initiator of the polymerization, at the incubation of the reaction compound in two stages, the first of which is conducted at the temperature of 20 - 90°C for 2 - 24 hours, **characterized by** the fact that the cross-linking agent is represented by a compound of N,N'-methylene-bis-acrylamide, N,N'-ethylene-bis-acrylamide and poviargolum with the following ratio of the components, in mass%:
Acrylamide - 1,3 - 15,
N,N'-methylene-bis-acrylamide - 0,004 - 0,975,
N,N'-ethylene-bis-acrylamide - 0,004 - 5,1,
poviargolum - 0,002 - 0,45,
water - up to 100,
the second stage of the reaction compound's incubation is conducted at the temperature of 107 - 130°C for not more than 2 hours.

11. The method, according to claim 10, **characterized in that** the cross-linking agent is additionally represented by vinylpyrrolydone and/or ethylene-bis-(oxyethylenenitrilo)-tetraacetic acid with the following ratio of the components, in mass%:
Acrylamide - 1,3 - 15,
N,N'-methylene-bis-acrylamide - 0,004 - 0,975,
N,N'-ethylene-bis-acrylamide - 0,004 - 5,1,
poviargolum - 0,002 - 0,45,
vinylpyrrolydone and/or ethylene-bis-(oxyethylene-nitrilo)-tetraacetic acid - 0,002 - 0,375, water - up to 100.

12. The method, according to claim 10, **characterized in that** after the first stage of the incubation the above-mentioned hydrogel is washed in hot water.

13. The method, according to claim 12, **characterized in that** the hydrogel is washed in water of 70 - 110°C for at least 3 hours.

14. The method, according to claim 12, **characterized in that** the washing of the hydrogel is carried out with 1 : 8 - 10 mass ratio of the hydrogel and water.

15. The method, according to claim 10, **characterized in that** the initiator of the polymerization is represented by hydrogen peroxide and/or ammonium persulfate in the amount not more than 0,3 mass% of the total weight of the original components.

16. The method, according to claim 10, **characterized in that** the aqueous medium is represented by bi-distilled apyrogenic water.

## Patentansprüche

1. Polyfunktionales biokompatibles Hydrogel, enthaltend ein vernetztes Kopolymer von Acrylamid mit einem Vernetzungsmittel und Wasser, **dadurch gekennzeichnet, dass** das Kopolymer eine Verbindung von N,N'-Methylen-bis-acrylamid, N,N'-Ethylen-bis-acrylamid und Poviargol als Vernetzungsmittel enthält.

2. Polyfunktionales biokompatibles Hydrogel, gemäß Anspruch 1, **dadurch gekennzeichnet, dass** das oben genannte vernetzte Kopolymer von Acrylamid mit einem Vernetzungsmittel das folgende Verhältnis von Bestandteilen, in Massen-%, enthält:
Acrylamid - 65,0 - 99,5,
N,N'-Methylen-bis-acrylamid - 0,2 - 6,5,
N,N'-Ethylen-bis-acrylamid - 0,2 - 34,0
Poviargol - 0,1 - 3,0.

3. Polyfunktionales biokompatibles Hydrogel, gemäß Anspruch 1, **dadurch gekennzeichnet, dass** es das folgende Verhältnis von Bestandteilen, in Massen-%, enthält:
Acrylamid - 1,3 - 15,
N,N'-Methylen-bis-acrylamid - 0,004 - 0,975,
N,N'-Ethylen-bis-acrylamid - 0,004 - 5,1
Poviargol - 0,002 - 0,45,
Wasser - auf 100.

4. Polyfunktionales biokompatibles Hydrogel, gemäß Anspruch 1, **dadurch gekennzeichnet, dass** das oben genannte vernetzte Kopolymer Vinylpyrrolidon und/oder Ethylen-bis-(oxyethylennitrilo)-tetraessigsäure als Vernetzungsmittel enthält.

5. Polyfunktionales biokompatibles Hydrogel, gemäß Anspruch 4, **dadurch gekennzeichnet, dass** das oben genannte vernetzte Kopolymer von Acrylamid mit einem Vernetzungsmittel das folgende Verhältnis von Bestandteilen, in Massen-%, enthält:
Acrylamid - 65,0 - 99,4,
N,N'-Methylen-bis-acrylamid - 0,2 - 6,5,
N,N'-Ethylen-bis-acrylamid - 0,2 - 34,0
Poviargol - 0,1 - 3,0,
Vinylpyrrolidon und/oder Ethylen-bis-(oxyethylennitrilo)-tetraessigsäure - 0,1 - 2,5.

6. Polyfunktionales biokompatibles Hydrogel, gemäß Anspruch 4, **dadurch gekennzeichnet, dass** es das folgende Verhältnis von Bestandteilen, in Massen-%, enthält:
Acrylamid - 1,3 - 15,
N,N'-Methylen-bis-acrylamid - 0,004 - 0,975,
N,N'-Ethylen-bis-acrylamid - 0,004 - 5,1
Poviargol - 0,002 - 0,45,
Vinylpyrrolidon und/oder Ethylen-bis-(oxyethylennitrilo)-tetraessigsäure - 0,002 - 0,375, Wasser - auf 100.

7. Polyfunktionales biokompatibles Hydrogel, gemäß Anspruch 1, **dadurch gekennzeichnet, dass** die Masse des oben genannten vernetzten Polymers von 2,0 bis 15 Massen-% der Gesamtmasse des oben genannten biokompatiblen Polymers beträgt.

8. Polyfunktionales biokompatibles Hydrogel, gemäß irgendeinem der Ansprüche 1 - 6, **dadurch gekennzeichnet, dass** es bi-destilliertes, pyrogenfreies Wasser enthält.

9. Polyfunktionales biokompatibles Hydrogel, gemäß irgendeinem der Ansprüche 1 - 7, **dadurch gekennzeichnet, dass** es einen pH-Wert von 3,5 - 7,5 hat.

10. Verfahren zur Herstellung des polyfunktionalen biokompatiblen Hydrogels mittels Kopolymerisation von Acrylamid mit einem Vernetzungsmittel im wässrigen Medium in Gegenwart eines Peroxid-Initiators der Polymerisation, durch Inkubation der Reaktionsverbindung in zwei Stufen, wobei die erste Stufe 2 - 24 Stunden lang bei der Temperatur von 20 - 90 °C ausgeführt wird, **dadurch gekennzeichnet, dass** das Vernetzungsmittel durch eine Verbindung von N,N'-Methylen-bis-acrylamid, N,N'-Ethylen-bis-acrylamid und Poviargol dargestellt ist, mit dem folgenden Verhältnis der Komponenten, in Massen-%:
Acrylamid - 1,3 - 15,
N,N'-Methylen-bis-acrylamid - 0,004 - 0,975,
N,N'-Ethylen-bis-acrylamid - 0,004 - 5,1
Poviargol - 0,002 - 0,45,
Wasser - auf 100,
die zweite Stufe der Inkubation der Reaktionsverbindung bei einer Temperatur von 107 - 130 °C für nicht länger als 2 Stunden durchgeführt wird.

11. Verfahren, gemäß Anspruch 10, **dadurch gekennzeichnet, dass** das Vernetzungsmittel zusätzlich durch Vinylpyrrolidon und/oder Ethylen-bis-(oxyethylennitrilo)-tetraessigsäure dargestellt ist, mit dem folgenden Verhältnis der Komponenten, in Massen-%:
Acrylamid - 1,3 - 15,
N,N'-Methylen-bis-acrylamid - 0,004 - 0,975,
N,N'-Ethylen-bis-acrylamid - 0,004 - 5,1
Poviargol - 0,002 - 0,45,
Vinylpyrrolidon und/oder Ethylen-bis-(oxyethylennitrilo)-tetraessigsäure - 0,002 - 0,375,
Wasser - auf 100.

12. Verfahren, gemäß Anspruch 10, **dadurch gekennzeichnet, dass** nach der ersten Stufe der Inkubation das oben genannte Hydrogel in heißem Wasser gewaschen wird.

13. Verfahren, gemäß Anspruch 12, **dadurch gekennzeichnet, dass** das Hydrogel wenigstens drei Stunden lang in Wasser von 70 - 110 °C gewaschen wird.

14. Verfahren, gemäß Anspruch 12, **dadurch gekennzeichnet, dass** das Waschen des Hydrogels in einem Massenverhältnis von 1 : 8 - 10 von Hydrogel zu Wasser durchgeführt wird.

15. Verfahren, gemäß Anspruch 10, **dadurch gekennzeichnet, dass** der Initiator der Polymerisation durch Wasserstoffperoxid und/oder Ammoniumpersulfat in einer Menge von nicht mehr als 0,3 Massen-% des Gesamtgewichts der Ausgangskomponenten dargestellt ist.

16. Verfahren, gemäß Anspruch 10, **dadurch gekennzeichnet, dass** das wässrige Medium durch bi-destilliertes, pyrogenfreies Wasser dargestellt ist.

## Revendications

1. Hydrogel biocompatible polyfonctionnel contenant un copolymère réticulé d'acrylamide avec un agent de réticulation et de l'eau, **caractérisé en ce que** ce copolymère comprend un composé de N,N'-méthylène-bis-acrylamide, de N,N'-éthylène-bis-acrylamide et de poviargolum en tant qu'agent de réticulation.

2. Hydrogel biocompatible polyfonctionnel selon la revendication 1, **caractérisé en ce que** le copolymère réticulé d'acrylamide mentionné ci-dessus avec un agent de réticulation contient la proportion suivante des composants, en % en masse :
acrylamide - 65,0-99,5,
N,N'-méthylène-bis-acrylamide - 0,2-6,5,
N,N'-éthylène-bis-acrylamide - 0,2-34,0,
poviargolum - 0,1-3,0.

3. Hydrogel biocompatible polyfonctionnel selon la revendication 1, **caractérisé en ce qu'**il contient la proportion suivante des composants, en % en masse :
acrylamide - 1,3-15,
N,N'-méthylène-bis-acrylamide - 0,004-0,975,
N,N'-éthylène-bis-acrylamide - 0,004-5,1,
poviargolum - 0,002-0,45,
eau - jusqu'à 100.

4. Hydrogel biocompatible polyfonctionnel selon la revendication 1, **caractérisé en ce que** le copolymère réticulé mentionné ci-dessus contient de la vinylpyrrolidone et/ou de l'acide éthylène-bis-(oxyéthylènenitrilo)-tétraacétique en tant qu'agent de réticulation.

5. Hydrogel biocompatible polyfonctionnel selon la revendication 4, **caractérisé en ce que** le copolymère réticulé d'acrylamide mentionné ci-dessus avec un agent de réticulation contient la proportion suivante des composants, en % en masse :
acrylamide - 65,0-99,4,
N,N'-méthylène-bis-acrylamide - 0,2-6,5,
N,N'-éthylène-bis-acrylamide - 0,2-34,0,
poviargolum - 0,1-3,0,
vinylpyrrolidone et/ou acide éthylène-bis-(oxyéthylènenitrilo)-tétraacétique - 0,1-2,5.

6. Hydrogel biocompatible polyfonctionnel selon la revendication 4, **caractérisé en ce qu'**il contient la proportion suivante des composants, en % en masse :
acrylamide - 1,3-15,
N,N'-méthylène-bis-acrylamide - 0,004-0,975,
N,N'-éthylène-bis-acrylamide - 0,004-5,1,
poviargolum - 0,002-0,45,
vinylpyrrolidone et/ou acide éthylène-bis-(oxyéthylènenitrilo)-tétraacétique - 0,002-0,375,
eau - jusqu'à 100.

7. Hydrogel biocompatible polyfonctionnel selon la revendication 1, **caractérisé en ce que** la masse du copolymère réticulé mentionné ci-dessus constitue de 2,0 à 15,0 % en masse de la masse totale de l'hydrogel biocompatible mentionné ci-dessus.

8. Hydrogel biocompatible polyfonctionnel selon l'une quelconque des revendications 1 à 6, **caractérisé en ce qu'**il contient de l'eau apyrogène bidistillée.

9. Hydrogel biocompatible polyfonctionnel selon l'une quelconque des revendications 1 à 7, **caractérisé en ce qu'**il présente un pH de 3,5 à 7,5.

10. Procédé de production de l'hydrogel biocompatible polyfonctionnel au moyen d'une copolymérisation de l'acrylamide avec un agent de réticulation dans un milieu aqueux en présence d'un amorceur peroxyde de la polymérisation, à l'incubation du composé de réaction en deux stades, dont le premier est réalisé à la température de 20 à 90 °C pendant de 2 à 24 heures, **caractérisé par le fait que** l'agent de réticulation est représenté par un composé de N,N'-méthylène-bis-acrylamide, de N,N'-éthylène-bis-acrylamide et de poviargolum avec la proportion suivante des composants, en % en masse :
acrylamide - 1,3-15,
N,N'-méthylène-bis-acrylamide - 0,004-0,975,
N,N'-éthylène-bis-acrylamide - 0,004-5,1,
poviargolum - 0,002-0,45,
eau - jusqu'à 100,
et le deuxième stade de l'incubation du composé de réaction est réalisé à la température de 107 à 130 °C pendant au plus 2 heures.

11. Procédé selon la revendication 10, **caractérisé en ce que** l'agent de réticulation est en outre représenté par de la vinylpyrrolidone et/ou de l'acide éthylène-bis-(oxyéthylènenitrilo)-tétraacétique avec la proportion suivante des composants, en % en masse :
acrylamide - 1,3-15,
N,N'-méthylène-bis-acrylamide - 0,004-0,975,
N,N'-éthylène-bis-acrylamide - 0,004-5,1,
poviargolum - 0,002-0,45,
vinylpyrrolidone et/ou acide éthylène-bis-(oxyéthylènenitrilo)-tétraacétique - 0,002-0,375,
eau - jusqu'à 100.

12. Procédé selon la revendication 10, **caractérisé en ce que** l'hydrogel mentionné ci-dessus est lavé dans de l'eau chaude après le premier stade de l'incubation.

13. Procédé selon la revendication 12, **caractérisé en ce que** l'hydrogel est lavé dans de l'eau à 70-110 °C pendant au moins 3 heures.

14. Procédé selon la revendication 12, **caractérisé en ce que** le lavage de l'hydrogel est réalisé avec un rapport massique entre l'hydrogel et l'eau de 1:8-10.

15. Procédé selon la revendication 10, **caractérisé en ce que** l'amorceur de la polymérisation est représenté par du peroxyde d'hydrogène et/ou du persulfate d'ammonium en une quantité d'au plus 0,3 % en masse du poids total des composants initiaux.

16. Procédé selon la revendication 10, **caractérisé en ce que** le milieu aqueux est représenté par de l'eau apyrogène bidistillée.
